(19) Europäisches Patentamt
European Patent Office
Office européen des brevets

(11) Veröffentlichungsnummer : **0 119 463 B2**

(12) **NEUE EUROPÄISCHE PATENTSCHRIFT**

(45) Veröffentlichungstag der neuen Patentschrift : **13.05.92 Patentblatt 92/20**

(51) Int. Cl.$^5$ : **C07C 51/487,** C07C 61/37, C07C 61/40, C07B 57/00, C07C 215/16

(21) Anmeldenummer : **84101504.3**

(22) Anmeldetag : **14.02.84**

# (54) Verfahren zur Trennung von substituierten Cyclopropancarbonsäureisomeren.

(30) Priorität : **17.02.83 HU 55183**

(43) Veröffentlichungstag der Anmeldung : **26.09.84 Patentblatt 84/39**

(45) Bekanntmachung des Hinweises auf die Patenterteilung : **23.09.87 Patentblatt 87/39**

(45) Bekanntmachung des Hinweises auf die Entscheidung über den Einspruch : **13.05.92 Patentblatt 92/20**

(84) Benannte Vertragsstaaten : **DE FR GB**

(56) Entgegenhaltungen :
**EP-A- 0 006 187**
**EP-A- 0 034 428**
**EP-B- 0 010 874**
**AGRICULTURAL AND BIOLOGICAL CHEMISTRY, Band 37, Nr. 7, 1973, Seiten 1713-1716, Tokyo, JP; F. HORIUCHI et al.: "New resolvingagents. 2-Benzylamino alcohols synthesized from natural amino acids"**

(73) Patentinhaber : **CHINOIN Gyogyszer és Vegyészeti Termékek Gyára RT.**
**To utca 1-5**
**H-1045 Budapest IV (HU)**

(72) Erfinder : **Fogassy, Elemér**
**Ördöngorom u. 20**
**H-1112 Budapest (HU)**
Erfinder : **Faigl, Ferenc**
**Körösi Cs. u. 9**
**H-1102 Budapest (HU)**
Erfinder : **So s, Rudolf**
**Hunyad lejt 12**
**H-1121 Budapest (HU)**
Erfinder : **Rák czi, J zsef**
**Fodor u. 18**
**H-1126 Budapest (HU)**
Erfinder : **Balogh, Gyula**
**Latinka S. u.15 c.1.**
**H-1116 Budapest (HU)**
Erfinder : **Boros Lászl né geb. Tudor, Irén**
**Lévai u. 31**
**H-2040 Budaörs (HU)**

(74) Vertreter : **Patentanwälte Beetz sen. - Beetz jun. Timpe - Siegfried - Schmitt-Fumian**
**Steinsdorfstrasse 10**
**W-8000 München 22 (DE)**



Jouve, 18, rue Saint-Denis, 75001 PARIS

**Beschreibung**

Die Erfindung betrifft ein neues Verfahren zur Trennung der vier Stereoisomeren von Vinylcyclopropancarbonsäuren der Formel I

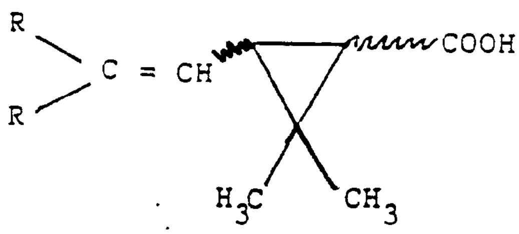

(I)

mit R = $CH_3$ oder Cl

Die Erfindung beruht auf der überraschenden Feststellung daß sich die D-trans, L-trans und D,L-cis Isomeren der cis,trans-Cyclopropancarbonsäuren besonders günstig mit den Enantiomeren D-N-Benzyl-2-aminobutanol und L-N-Benzyl-2-aminobutanol trennen lassen.

Weiterhin wurde gefunden, daß die racemischen cis- und die racemischen trans-Cyclopropancarbonsäuren in bestimmten Lösungsmitteln große Unterschiede in der Löslichkeit zeigen, und sich deshalb unter bestimmten Bedingungen durch ein einfaches Verfahren in technischem Maßstab praktisch quantitativ trennen lassen. Die Enantiomeren der erhaltenen cis- und trans-Racemate können mit den Enantiomeren des N-Benzyl-2-aminobutanols mit großer Selektivität getrennt werden.

Die Verbindungen der Formel I sind Produkte bzw. Zwischenprodukte der Synthese von insekticid wirksamen Pyrethroiden. Die biologische Wirksamkeit der Pyrethroide hängt stark von der Raumstruktur der bei der Synthese verwendeten Carbonsäure der Formel I ab. Die Trennung der optischen Isomeren der obigen Carbonsäuren mit gutem Wirkungsgrad ist demzufolge von großer Bedeutung.

Es ist bereits ein Verfahren zur Trennung der Isomeren der racemischen cis- und trans-2,2-Dimethyl-3-(2,2-dichlorvinyl)-cyclopropancarbonsäure (Permethrinsäure) durch mehrmalige Umkristallisation aus n-Hexan (Coll. Czech. Chem. Commun. 24, 2230) bekannt, wobei durch die Anreicherung der D,L-trans-Permethrinsäure in der Kristallphase eine Ausbeute von ca. 30 % erreicht wird. Nach der DE-PS 2 716 898 werden die cis- und trans-Isomeren der Cyclopropancarbonsäuren durch fraktionierte Destillation getrennt. Die DE-PS 2 713 538 beschreibt ein Verfahren zur Trennung der Stereoisomeren von substituierten Cyclopropancarbonsäuren durch Extraktion ihrer wässerigen Lösung mit apolaren Lösungsmitteln. Ferner sind mehrere Verfahren zur Trennung der Isomeren der racemischen cis- und trans-2,2-Dimethyl-3-(2-methylpropen-1-yl)-cyclopropancarbonsäure (Chrysanthemumsäure) bekanntgeworden. So ist z. B. ein Verfahren zur Trennung der Isomeren durch Kristallisation aus Petrolether in Helv. Chim. Acta 7, 396 und durch Kristallisation aus Ethylacetat in J. Am. Chem. Soc. 1945, 238 beschrieben.

Die Nachteile der bekannten Verfahren bestehen darin, daß zur Herstellung von reinen cis- und trans-Isomeren mehrere Umkristallisationen mit großem Substanzverlust durchgeführt werden müssen.

Auch zur Trennung der optischen Isomeren der Cyclopropancarbonsäuren der Formel I sind zahlreiche Verfahren angegeben worden. DE-A-2 826 952 beschreibt ein Verfahren zur Gewinnung der trans-Permethrinsäure mit (-)-Phenylglycinethylester. Als Trennmittel können auch die optisch aktiven Enantiomeren von Naphthylethylamin, Cinchonidin, Chinin, N,N-Dimethyl-2-amino-1-p-nitrophenylpropan-1,3-diol verwendet werden (HU-A-158 498, GB-A-1 369 730, 1 226 914 und 1 178 423). Diese Verfahren sind wegen der teuren Trennmittel und aufwendigen Trennverfahren nachteilig, wobei meistens nur ein Racemat, das cis- oder das trans-Enantiomer zugänglich sind. Zur Erhöhung der optischen Reinheit der Enantiomeren müssen teure und mit großem Substanzverlust verbundene Umkristallisationen durchgeführt werden. Eine Regenerierung des Trennmittels wurde bisher nicht beschrieben.

Aus EP-A-10874 ist ein Verfahren zur Abtrennung der einzelnen optisch aktiven Isomeren von cis- oder trans-Cyclopropancarbonsäuren, die in 3-Stellung mit einer Alkenylgruppe substituiert sein können, aus Gemischen entsprechender cis- oder trans-Isomerer bekannt, bei dem eine wäßrige Lösung eines löslichen Salzes, z.B. eines Alkalisalzes, des Racemats oder Diastereomerengemisches der cis- oder trans-Isomeren mit einem Isomer eines optisch aktiven Amins umgesetzt wird, wobei ein entsprechendes diastereomeres Ammoniumsalz ausfällt, das abgetrennt wird, und die Gesamtmenge des anderen Isomers bzw. der anderen Isomeren in Lösung bleibt und daraus gewonnen werden kann.

In dieser Druckschrift ist als optisch aktives Amin lediglich 1-α-Methylbenzylamin genannt. Der mögliche Alkenyl-substituent ist ferner in einer Aufzählung mehrerer Substituentenbedeutungen genannt.

Aus Agricultural and Biological Chemistry, 37 (7) (1973) 1713 - 1716, ist die Trennung von D,L-trans-Chrysanthemumsäure mit verschiedenen optisch aktiven 2-N-Benzylaminoalkoholen bekannt. Die erfindungsgemäß verwendete Verbindung 2-N-Benzylaminobutanol ist unter den in dieser Druckschrift aufgelisteten Diastereomerenpaarbildnern nicht aufgeführt.

Bei der Trennung optischer Isomerer übt sogar eine geringe Modifizierung der Struktur der Reagentien einen starken Einfluß auf das Trennergebnis aus. Die Eigenschaften der bei der Trennung entstehenden Diastereomerenpaare, welche die Trennung ermöglichen, wie z. B. deren Löslichkeit, Dissoziationsfähigkeit udgl., sind ferner stark vom verwendeten Reagens abhängig.

Ein weiterer wesentlicher Unterschied besteht in der Anwendbarkeit der Verfahren. Die oben erwähnte, dem Stand der Technik entsprechende Druckschrift beschreibt lediglich die Auftrennung racemischer trans-Chrysanthemumsäure. Auf der anderen Seite ist das erfindungsgemäße Verfahren zur Trennung von racemischen cis- und racemischen trans-Isomeren und Isomerengemischen der Chrysanthemumsäure und deren Halogenderivaten, wie etwa der Permethrinsäure, Decamethrinsäure etc., ebenso gut geeignet.

Die technischen Verfahren zur Synthese der Cyclopropancarbonsäuren der Formel I betreffen meistens ein D,L-cis,trans-Isomerengemisch. Die Trennung der vier Isomeren, um Pyrethroide mit höherer Wirksamkeit zu erzielen, hat unter gewerblichen und ökonomischen Gesichtspunkten große Bedeutung. Diese Anforderungen können durch das erfindungsgemäße Verfahren erfüllt werden.

Der Erfindung liegt die Aufgabe zugrunde, neuartige Verfahren zur Trennung der optisch aktiven cis- und trans-Isomeren von Vinylcyclopropancarbonsäurederivaten in die entsprechenden vier Stereoisomeren anzugeben.

Die Aufgabe wird anspruchsgemäß gelöst. Vorteilhafte Ausführungsformen sind Gegenstand der abhängigen Ansprüche.

Das zugrundeliegende allgemeine Verfahrensprinzip zur Gewinnung der optisch aktiven cis- und trans-Isomer von Alkenylcyclopropancarbonsäurederivaten durch Trennung von D,L-cis, trans-, D,L-cis- und D,L-trans-Alkenylcyclopropancarbonsäurederivaten beruht auf

– Umsetzung der entsprechenden Isomerengemische in Form löslicher Alkalimetallsalze mit einem Isomer eines optisch aktiven Amins in einer wäßrigen Lösung unter Ausfällung eines kristallinen diastereomeren Ammoniumsalzes,

– Abtrennung dieses diastereomeren Ammoniumsalzes,

– Überführung durch Ansäuern mit einer Mineralsäure in das entsprechende optisch aktive Alkenylcyclopropancarbonsäurederivat,

– Absaugen dieses Derivats

und

– Gewinnung des anderen Isomers oder Isomerengemischs durch Ansäuern des erhaltenen Filtrats mit einer Mineralsäure

sowie gegebenenfalls

– Reinigung des erhaltenen optisch aktiven Isomers der Verbindung der Formel I durch selektive Abscheidung und

– Regenerierung des eingesetzten optisch aktiven Amins; es ist dadurch gekennzeichnet, daß

– (a) Salze von D,L-cis,trans-Vinylcyclopropancarbonsäurederivaten der Formel I

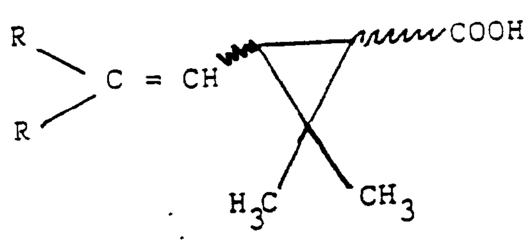

(I)

mit R = $CH_3$ oder Cl

in beliebigem Isomerenverhältnis

oder

– (b) durch selektives Lösen in apolaren aromatischen oder aliphatischen Kohlenwasserstoffen als Lösungsmitteln hergestellte reine , Formel I entsprechende D,L-cis- und D,L-trans-Vinylcyclopropancarbonsäurederivate,

die mit Alkalihydroxiden oder Alkalicarbonaten in entsprechende Salze übergeführt wurden,

eingesetzt werden,

– als Amin 2-N-Benzylaminobutanol oder das Hydrochlorid davon verwendet wird

und

– die Umsetzung in Wasser oder einem Wasser-Aceton- Gemisch durchgeführt wird.

Die Erfindung beruht auf der Feststellung, daß sich die Enantiomeren aus beliebigen Gemischen von Isomeren der Cyclopropancarbonsäuren der Formel I besonders erfolgreich durch die Enantiomeren des N-Benzyl-2-aminobutanols trennen lassen. So ergibt sich z. B. ein kristallines Diastereomersalz der D-trans-Permethrinsäure aus einer Lösung von racemischer cis,trans-Permethrinsäure in Wasser mit D-N-Benzyl-2-aminobutanol in Gegenwart einer entsprechenden Menge an Alkalihydroxiden oder Alkalicarbonaten, wenn das Trennmittel in diesem Enantiomer in äquivalenter Menge zugefügt wird. Aus der im Filtrat zurückgebliebenen L-trans-D,L-cis-Permethrinsäure kann auch das L-trans-Isomer gewonnen werden, wenn der Lösung L-N-Benzyl-2-aminobutanol in diesem Enantiomer in äquivalenter Menge zugefügt wird. Es wurde weiterhin gefunden, daß sich die optischen Isomeren der zurückbleibenden D,L-cis-Permethrinsäure mit guter Ausbeute durch die Enantiomeren des N-Benzyl-2-aminobutanols trennen lassen. Hier ergibt sich jedoch ein kristallines Diastereomersalz der L-cis-Permethrinsäure mit D-N-Benzyl-2-aminobutanol, wenn die Auftrennung unter basischen Bedingungen, vorzugsweise bei pH $\geqq$ 8 - 8,5, durchgeführt wird.

Das Wesen der Erfindung besteht darin, daß das oben erwähnte Trennmittel besonders erfolgreich zur Trennung der reinen, optischen Isomeren sowohl der D,L-cis und D,L-trans-Permethrinsäuren als auch der D,L-cis- und D,L-trans-Chrysantemumsäuren verwendbar ist. Der Vorteil der Erfindung besteht darin, daß die Auftrennung sämtlicher Racemate unter fast gleichen Bedingungen in wäßriger Lösung durchgeführt werden kann. Dies bedeutet ein einfaches, wirkungsvolles und flexibleres Verfahren.

Zur Trennung können die D,L-cis- und D,L-trans-Permethrinsäuren bzw. die D,L-cis und D,L-trans-Chrysanthemumsäuren gegebenenfalls aus ihren cis-trans-Mischungen aufgrund der unterschiedlichen Löslichkeit der Isomeren in bestimmten Lösungsmitteln hergestellt werden.

Es wurde ferner gefunden, daß das Mengenverhältnis der cis,trans-Isomeren in der gewünschten Richtung und im gewünschten Maße praktisch unabhängig vom Anfangszustand verändert werden kann.

Soll die relative Menge von D,L-cis-Permethrinsäure erhöht werden, so wird dies durch Vermischen mit der entsprechenden Menge Benzol bei einer Temperatur von 20 bis 30°C erreicht. Bei Rühren bei dieser Temperatur während einer entsprechenden Zeitdauer stellt sich ein Lösungsgleichgewicht ein, wobei das Verhältnis der in Benzol schlecht löslichen D,L-cis-Permethrinsäure im Filtrat nur von der Menge des Lösungsmittels abhängig ist. Durch Erhöhung des Verhältnisses Benzol/Permethrinsäure kann der Gehalt des cis-Isomers in der festen Phase deutlich erhöht werden. Die durch das Eindampfen der Lösungen erhaltenen Rückstände, die große Mengen von trans-Isomeren enthalten, weisen ein gleichbleibendes Isomerenverhältnis auf, wodurch sie vereinigt und gegebenenfalls zur Herstellung von reinem D,L-trans-Isomer verwendet werden können. Zur Herstellung der reinen D,L-trans-Permethrinsäure wird der obige Rückstand, der eine große Menge trans-Isomer enthält, mit apolaren Lösungsmitteln, wie Extraktionsbenzin oder n-Hexan, vermischt. Die relative Menge der D,L-trans-Permethrinsäure im Filtrat ändert sich im direkten Verhältnis zur Menge des als Lösungsmittel zur Gewichtseinheit des Isomerengemisches verwendeten aliphatischen Kohlenwasserstoffes. Die aus den Filtraten isolierten Stoffe weisen gleichzeitig ein gleichbleibendes Isomerenverhältnis auf, wodurch sie vereinigt und zur weiteren Trennung verarbeitet werden können. Ein Isomerengemisch von cis- und trans-Permethrinsäure mit beliebiger Zusammensetzung kann also durch alternative Verwendung eines aromatischen Lösungsmittels, vorzugsweise Benzol, und eines aliphatischen Kohlenwasserstoff-Lösungsmittels, vorzugsweise Extraktionsbenzin oder n-Hexan, praktisch quantitativ in die D,L-cis- und die D,L-trans-Isomeren getrennt werden. Sollen die cis- und trans-Isomeren der D,L-cis,trans-Chrysanthemumsäure (R = Methyl in Formel I) getrennt werden, so kann dies aufgrund der großen Unterschiede in der Löslichkeit dieser Isomeren leicht erreicht werden. Da diese Stoffe ihre Löslichkeit gegenseitig nicht beeinflussen, kann die Verwendung eines aromatischen Lösungsmittels gegebenenfalls entfallen. Das cis-Isomer kann aus der Festsubstanz gewonnen werden, die nach Vermischen mit einem aliphatischen Kohlenwasserstoff-Lösungsmittel, vorzugsweise n-Hexan, als Rückstand verbleibt.

Die Herstellung des trans-Isomers erfolgt durch Einengen des Filtrats und erneute Behandlung des Rückstands mit einem aliphatischen Kohlenwasserstoff-Lösungsmittel. Durch mehrmalige Verwendung der Lösungen kann auch in diesem Fall eine quantitative Trennung erreicht werden.

Eine andere Möglichkeit zur Herstellung von D,L-trans-Chrysanthemumsäure beruht darauf, daß die Säureisomeren und ihre Natriumsalze eine entgegengesetzte Löslichkeit besitzen. Zur Trennung wird die Isomerenmischung in Wasser/Aceton zum Na-Salz umgesetzt, worauf das D,L-trans-Salz abgesaugt und die Säure auf bekannte Weise freigesetzt wird.

Nach dem erfindungsgemäßen Verfahren werden die erhaltenen D,L-cis- und D,L-transpermethrinsäuren und -Chrysanthemumsäuren mit den Enantiomeren des N-Benzyl-2-aminobutanols getrennt, wobei das Racemat der Säure mit 1,0 bis 1,3 Äquivalent Alkalihydroxid oder Alkalicarbonat in Wasser gelöst und mit einer Lösung von 0,4 bis 0,6 Äquivalent Trennmittel in Aceton oder dessen Hydrochloriden in Wasser/Aceton, vorzugsweise bei einer Temperatur von 40 bis 90°C, versetzt wird. Nach Kühlung wird das erhaltene kristalline Diastereomersalz abgesaugt.

Wenn als Trennmittel D-N-Benzyl-2-aminobutanol verwendet wird, so kann man aus D,L-trans-Permethrinsäure das D-trans-Isomer, aus D,L-cis-Permethrinsäure das L-cis-Enantiomer, aus D,L-trans-Chrysanthemumsäure die D-trans-Chrysanthemumsäure und aus D,L-cis-Chrysanthemumsäure die L-cis-Chrysanthemumsäure herstellen, wobei immer ein kristallines Diastereomersalz mit dem Trennmittel ausgeschieden wird. Mit L-N-Benzyl-2-aminobutanol kann immer die entgegengesetzte Antipodenverbindung kristallisiert werden.

Die erwähnten Diastereomersalze sind neue Verbindungen, die in der Literatur bisher nicht beschrieben worden sind.

Die optische Reinheit der Enantiomeren in den Diastereomersalzen ist von der Wasserstoffionenkonzentration des Mediums bei der Trennung abhängig. Im Hinblick auf den Trennungswirkungsgrad des Verfahrens ist also wichtig, die Menge der Base, die zum Lösen der racemischen Säuren verwendet wird, und die Menge der Säure, die gegebenenfalls zum Lösen des Trennmittels verwendet wird, genau einzustellen. Zur optimalen Trennung der Isomeren der Cyclopropancarbonsäuren der Formel I werden 1,0 bis 1,3 Äquivalent und vorzugsweise 1,2 Äquivalent Base, bezogen auf die racemische Säure, verwendet.

Es wurde ferner gefunden, daß die optische Reinheit der durch das Trennmittel hergestellten optisch aktiven cis- und trans-Permethrin- und Chrysanthemumsäuren durch selektive Ausscheidung erhöht werden kann. Die erhaltenen optisch aktiven Carbonsäuren werden mit Alkalihydroxid gelöst und mit Mineralsäure in kleinerer als aufgrund der optischen Reinheit der Säure berechneter äquivalenter Menge bei einer Temperatur von 0 bis 10°C versetzt. Die Fraktion der Säure, die kristallin ausfällt, und die Fraktion, die gelöst bleibt, weisen gegenseitig sowie bezüglich der Ausgangsreinheit eine unterschiedliche optische Reinheit auf. Mit dieser Methode kann man erreichen, daß eine Fraktion nur Racemat und die andere nur optisch reine Carbonsäure enthält Die Fraktion mit dem Racemat kann zur Trennung rückgeführt werden.

In wirtschaftlicher Hinsicht kann das erfindungsgemäße Verfahren dadurch verbessert werden, daß das verwendete Trennmittel aus den sauren und wässerigen Lösungen, die bei der Trennung anfallen, leicht und quantitativ regeneriert werden kann.

Das erfindungsgemäße Verfahren wird durch die folgenden Beispiele näher erläutert.

**Beispiel 1**

a) 10,5 g Isomerengemisch von racemischer cis,trans-Permethrinsäure (enthaltend 40 % cis- und 60% trans-Isomer) löst man in 60 ml Wasser mit Gehalt von 2,4 g Natriumhydroxid und erhitzt auf 60°C. Bei dieser Temperatur fügt man eine Lösung von 2,3 g D-N-Benzyl-2-aminobutanol in 10 ml Aceton zu. Man läßt die Mischung unter Rühren abkühlen und impft mit 0,1 g D-trans-Permethrinsäure-D-N-benzyl-2-aminobutanolsalz. Man läßt das Gemisch 3 Stunden bei 25°C stehen, saugt schließlich das Produkt ab und wäscht zweimal mit je 5 ml Wasser. Man erhält so 3,5 g Salz. Man suspendiert das Salz in einer Mischung von 10 ml Wasser und 20 ml Chloroform, kühlt auf 10°C ab und säuert bis auf pH 1 mit 5 N Salzsäure an. Man trennt die erhaltenen zwei Phasen nach 15 min Stehen, extrahiert die wässerige Phase mit 10 ml Chloroform, trocknet die vereinigten Chloroformphasen über Natriumsulfat und saugt ab. Man dampft dann die Lösungsmittel aus dem Filtrat im Vakuum ab und erhält als Rückstand 1,85 g D-trans-Permethrinsäure (63 %; $[\alpha]_D^{25}$ + 34° (c = 1,8, Chloroform); Fp. 53-61°C).

Man engt das Filtrat der Trennstufe auf ein Drittel ein, verdünnt den Rückstand mit 10 ml Wasser und säuert mit 5 N Salzsäure bis auf pH 1 an. Man extrahiert dann das entstandene Öl zweimal mit je 20 ml Chloroform, trocknet die organischen Phasen über Natriumsulfat und saugt ab. Man engt das Filtrat ein und erhält als Rückstand 8 g L-trans-D,L-cis-Permethrinsäure ($[\alpha]_D^{25}$ -6,7° (c = 2, Chloroform), Fp. 53-65°C).

b) Man arbeitet analog Verfahren a), jedoch mit dem Unterschied, daß man zur Lösung der racemischen Permethrinsäure 4,2 g Natriumhydrogencarbonat anstelle von Natriumhydroxid verwendet. Aus dem Salz erhält man 1,65 g D-trans-Permethrinsäure ($[\alpha]_D^{25}$ +33° (c = 2, Chloroform)). Aus dem Filtrat der Trennstufe erhält man 7 g L-trans-D,L-cis-Permethrinsäure ($[\alpha]_D^{25}$ -7° (c = 2, Chloroform)).

c) Man arbeitet analog Verfahren a), jedoch mit dem Unterschied, daß man zur Lösung der racemischen Permethrinsäure 5,3 g Natriumcarbonat anstelle von Natriumhydroxid verwendet. Aus dem Salz erhält man 2,0 g D-trans-Permethrinsäure ($[\alpha]_D^{25}$ + 38° (c = 2, Chloroform)). Aus dem Filtrat der Trennstufe erhält man 7,5 g L-trans- D,L-cis-Permethrinsäure ($[\alpha]_D^{25}$ -7,8° (c = 1,6, Chloroform)).

**Beispiel 2**

a) 100 g Isomerengemisch der D,L-cis,trans-Permethrinsäure (cis/trans-Isomerenverhältnis 40:60) rührt man in 400 ml Benzol 1 Stunde bei 27°C. Man saugt dann die Suspension ab und erhält 31 g Permethrinsäure mit einem cis/trans-Isomerenverhältnis von 79:21, die zur Herstellung von racemischer cis-Permethrinsäure analog dem weiter unten beschriebenen Beispiel 4 b) aufgearbeitet wird. Man dampft die Benzollösung ab und extrahiert den erhaltenen Rückstand (67,2 g), der 23 % cis- und 77 % trans-Isomer enthält, 5 Stunden bei 30°C mit 240 ml Extraktionsbenzin unter Rühren. Man saugt dann die erhaltene Suspension ab und erhält als Feststoff 26,8 g D,L-trans-Permethrinsäure mit einem trans-Isomergehalt über 95 %.

Man befreit dann das Benzinfiltrat vom Lösungsmittel durch Eindampfen und erhält 40 g Isomermischung der Permethrinsäure mit einem cis/trans-Verhältnis von 38:62, die als Ausgangsmaterial zur Trennung der D,L-cis und D Ltrans-Isomeren verwendet werden kann.

b) Man löst 10,5 g D,L-trans-Permethrinsäure gemäß Beispiel 2a) in einer Lösung von 2,2 g Natriumhydroxid in 60 ml Wasser. Man erwärmt die Lösung auf 70°C und fügt eine Lösung von 4,5 g D-N-Benzyl-2-aminobutanol in einer Mischung aus 20 ml Aceton, 10 ml Wasser und 2,5 ml 10 N Salzsäure hinzu. Man kühlt den Ansatz unter Rühren auf 25° C ab und läßt ihn 10 Stunden bei dieser Temperatur stehen. Man saugt danach die Kristalle ab, wäscht zweimal mit je 5 ml Wasser, trocknet und erhält 6,0 g Diastereomerensalz.

Man suspendiert das Salz in einer Mischung von 10 ml Wasser und 20 ml Chloroform und säuert mit 5 N Salzsäure bis auf pH 1 an. Man trennt die organische Phase ab und extrahiert die wässerige Phase mit 10 ml Chloroform. Man vereinigt anschließend die Chloroformphasen, trocknet über Natriumsulfat, befreit vom Lösungsmittel durch Eindampfen und erhält 3,1 g D-trans-Permethrinsäure ($[\alpha]_D^{25}$ + 35° (c = 2,5, Chloroform)). Man engt das Filtrat im Vakuum ein; durch übliche Aufarbeitung mit Salzsäure erhält man 7,2 g L-trans-Permethrinsäure ($[\alpha]_D^{25}$ -17,5° (c = 2, Chloroform)).

c) Man löst 10,5 D,L-trans-Permethrinsäure in einer Lösung von 2,4 g Natriumhydroxid in 50 ml Wasser, erwärmt auf 80°C und fügt eine Lösung von 4,5 g D-N-Benzyl-2-aminobutanol in 2,5 ml 10 N Salzsäure und 20 ml Wasser hinzu. Analog Beispiel 2b) erhält man 4,0 g D-trans-Permethrinsäure. Da die Trennlösung kein Aceton enthält, erhält man aus dem Filtrat ohne Eindampfen durch Ansäuern bis pH 1 6,0 g L-trans-Permethrinsäure ($[\alpha]_D^{25}$ -13,5° (c = 1,9; Chloroform)).

**Beispiel 3**

a) Man rührt 100 g D,L-cis,trans-Chrysanthemumsäure mit einem cis/trans-Isomerenverhältnis von 40:60 1 h bei 25°C in 45 ml n-Hexan. Man saugt die Suspension ab und erhält als festen Stoff 35,3 g Chrysanthemumsäure mit einem cis/trans-Isomerenverhältnis von 80:20. Durch Eindampfen des Filtrats erhält man 62 g Chrysanthemumsäure mit einem cis/trans-Isomerenverhältnis von 18:82, die mit 35 ml n-Hexan bei 25°C 30 min gerührt und dann abgesaugt wird. Man engt das Filtrat auf die Hälfte ein und rührt 1 h bei 0°C. Man saugt dann die erhaltenen Kristalle ab und wiederholt die obige Ausrührung. So erhält man 9,1 g D,L-trans-Chrysanthemumsäure, die 99,8 % trans-Isomer enthält; Fp. 52 - 54° C.

b) Man löst 3,36 g D,L-trans-Chrysanthemumsäure in einer Lösung von 0,96 g Natriumhydroxid in 15 ml Wasser, erwarmt auf 60°C und fügt eine Lösung von 1,8 g D-N-Benzyl-2-aminobutanol in einer Mischung von 5 ml 5 N Salzsäure und 10 ml Wasser hinzu. Man läßt den Ansatz auf 25°C abkühlen, saugt die Kristalle nach 2 h Stehen ab, wäscht zweimal mit je 2 ml Wasser und erhält 3,5 g Diastereomersalz. Man suspendiert das Salz in einer Mischung von 10 ml Wasser und 20 ml Chloroform und säuert mit 5 N Salzsäure bis pH 1 an. Man trennt danach die organische Phase ab, trocknet über Natriumsulfat, befreit vom Chloroform durch Eindampfen in Vakuum und erhält 1,6 g D-trans-Chrysanthemumsäure ($[\alpha]_D^{25}$ 20° (c = 1,8, Chloroform)).

Man engt das Filtrat der Trennstufe im Vakuum auf die Hälfte ein, verdünnt mit 10 ml Wasser und säuert mit 5 N-Salzsäure bis pH 1 an. Man extrahiert dann das entstandene Öl mit 20 ml Chloroform, trocknet die organische Phase über Natriumsulfat, engt ein und erhält 1,6 g L-trans-Chrysanthemumsäure ($[\alpha]_D^{25}$ -20° (c = 2, Chloroform)).

**Beispiel 4**

a) Man rührt 100 g D,L-cis,trans-Permethrinsäure mit einem cis/trans-Isomerenverhältnis von 40:60 in 500 ml Benzol 5 h bei 30°C und saugt dann die Suspension ab. Der erhaltene Feststoff (23 g) enthält 97,5 % cis-Isomer. Gewünschtenfalls kristallisiert man aus 70 ml Benzol um und erhält 18,4 g D,L-cis-Permethrinsäure mit einem cis-Isomergehalt von 99,8 %; Fp. 86,9 - 88°C. Man engt das Filtrat der Benzolextraktion ein und erhält 75 g Permethrinsäure mit einem cis/trans-Isomerenverhältnis von 24:76, die zur Herstellung von D,L-trans-Isomer nach Beispiel 2 verwendet werden kann.

b) Man rührt 31,0 g Permethrinsäure mit einem cis/trans-Isomerenverhältnis von 79:21, hergestellt nach Beispiel 2a), 5 h in 120 ml Benzol bei 27°C und saugt dann die Suspension ab. Der erhaltene Feststoff (27,7 g) enthält 98,1 % cis-Isomer. Gewünschtenfalls kristallisiert man aus 70 ml Benzol um und erhält 18,1 g D,L-cis-Permethrinsäure (99,8 %).

Man engt das Benzolfiltrat ein und erhält 2,6 g Permethrinsäure mit einem cis/trans-Isomerenverhältnis von 24:76, die zur Herstellung von D,L-trans-Isomer nach Beispiel 2 verwendet werden kann.

c) Man gibt 10,5 g D,L-cis-Permethrinsäure, hergestellt nach Beispiel 4a) bzw. 4b), zu einer Lösung von 2,2 g Natriumhydroxid in 50 ml Wasser und erwärmt auf 60°C. Zu dieser Lösung fügt maneine Lösung von 4,5 g D-N-Benzyl-2-aminobutanol in einer Mischung von 10 ml Wasser, 10 ml Aceton und 2,5 ml 10 N Salzsäure hinzu. Die Ausscheidung der Kristalle beginnt unter Kühlung. Das kristalline Salz saugt man nach 10 h Stehen ab, wäscht zweimal mit je 5 ml Wasser und erhält 4,0 g Salz.

Man suspendiert das Salz in einer Mischung von 10 ml Wasser und 20 ml Chloroform, trennt die organische Phase nach Ansäuerung auf pH 1 ab, trocknet über Natriumsulfat und engt ein. (Falls die Ansäuerung bei einer Temperatur unter +10°C durchgeführt wurde, kann die Extraktion weggelassen werden, da das Produkt kristallin anfällt). Auf diese Weise erhält man 2,3 g L-cis-Permethrinsäure ($[\alpha]_D^{25}$ - 24° (c = 2,1, Chlo-Chloroform), Fp. 82-85° C).

Man engt das Filtrat der Trennstufe im Vakuum auf ein Drittel ein, verdünnt den Rückstand mit 10 ml Wasser, säuert mit 5 N Salzsäure bei einer Temperatur von + 10° C bis auf pH 1 an, saugt die Kristalle nach 1 h Stehen ab, wäscht dreimal mit je 5 ml Wasser und erhält 8,0 g D-cis-Permethrinsäure ($[\alpha]_D^{25}$ +4,2° (c = 1,6, Chloroform), Fp. 78 - 82° C).

**Beispiel 5**

a) Man rührt 35,3 g cis-Chrysanthemumsäure mit einem cis-Isomergehalt von 80 %, hergestellt nach Beispiel 3a), in 50 ml n-Hexan 30 min bei 25°C und saugt dann ab.

Die erhaltenen Kristalle wäscht man mit 10 ml n-Hexan bei 0 bis 5°C und kristallisiert ohne Trocknen aus 75 ml Methanol um. Auf diese Weise erhält man 20,4 g D,L-cis-Chrysanthemumsäure, die 99,8 % cis-Isomer enthält; Fp. 114-116° C.

Man trennt die an trans-Isomeren reiche Cyclopropancarbonsäure aus den Hexanlösungen durch Eindampfen ab und führt sie zur Isomerentrennung zurück.

b) Man gibt 3,36 g D,L-cis-Chrysanthemumsäure, hergestellt nach Beispiel 5a), zu einer Lösung von 0,9 g Natriumhydroxid in 15 ml Wasser zu, erwärmt auf 65°C und versetzt mit einer Lösung von 1,79 g DN-Benzyl-2-aminobutanol in einer Mischung aus 5 ml Wasser und 5 ml 2 N Salzsäure. Man läßt den Ansatz auf 25°C abkühlen und saugt dann das kristalline Diastereomersalz nach 2 h Stehen ab. Auf diese Weise erhält man 3,3 g Salz, das in 10 ml Wasser und 20 ml Chloroform suspendiert und mit 5 N Salzsäure bis auf pH 1 angesäuert wird. Man trennt dann die organische Phase ab, trocknet über Natriumsulfat, engt ein und erhält 1,6 g L-cis-Chrysanthemumsäure ($[\alpha]_D^{25}$ - 50° (c = 2 Chloroform)).

Aus dem Filtrat der Trennstufe erhält man analog der Salzaufarbeitung 1,4 g D-cis-Chrysanthemumsäure ($[\alpha]_D^{25}$ + 47° (c = 2,1, Chloroform)).

**Beispiel 6**

Man gibt 8,0 g L-trans-D,L-cis-Permethrinsäure, hergestellt nach Beispiel 1a), zu einer Lösung von 1,6 g Natriumhydroxid in 60 ml Wasser, erwärmt auf 70°C und fügt eine Lösung von 2,6 g L-N-Benzyl-2-aminobutanol in 7 ml Wasser hinzu. Man läßt den Ansatz abkühlen und läßt dann bei 25°C weitere 2 h stehen. Das ausgeschiedene L-trans-Permethrinsäure-L-N-benzyl-2-aminobutanolsalz saugt man ab und wäscht zweimal mit je 5 ml Wasser. Auf diese Weise erhält man 4,0 g Salz.

Nach Aufarbeitung des Salzes nach Beispiel 1a) erhält man 2,0 g L-trans-Permethrinsäure ($[\alpha]_D^{25}$ -30° (c = 2, Chloroform)).

Das Filtrat der Trennstufe säuert man mit 5 N Salzsäure bis auf pH 1 an. Man extrahiert das ausgeschiedene Öl mit Chloroform, trennt die organische Phase ab, trocknet über Natriumsulfat und dampft ein. Der Rückstand ist 5,2 g D,L-Permethrinsäure mit hohem Gehalt an cis-Isomer, aus der die Enantiomeren analog Beispiel 4 hergestellt werden können.

**Beispiel 7**

a) Man löst 6,0 g L-trans-Permethrinsäure ($[\alpha]_D^{25}$ -17,5° (c = 2, Chloroform)) in einer Mischung von 12 ml 10 %igem Natriumhydroxid und 5 ml Wasser, kühlt auf +10°C ab und fügt 5 ml 2 N Salzsäure hinzu. Man saugt die ausgefallenen Kristalle ab und erhält 2,3 g Produkt (- 25° (c = 2, Chloroform)). Man säuert das Filtrat bis auf pH 1 an und saugt das ausgeschiedene Produkt ab. Auf diese Weise erhält man 3,4 g Produkt ($[\alpha]_D^{25}$ -8,0° (c = 2,1, Chloroform)).

b) Man löst 2,0 g L-trans-Permethrinsäure ($[\alpha]_D^{25}$ -25° (c = 2, Chloroform)) in einer Mischung von 4 ml 10 %igem Natriumhydroxid und 1,6 ml Wasser, kühlt auf + 10°C ab und fügt 1,6 ml 2 N Salzsäure hinzu. Man saugt die ausgeschiedenen Kristalle ab und erhält 0,75 g Produkt ($[\alpha]_D^{25}$ -38° (c = 2, Chloroform)).

Man säuert das Filtrat bis auf pH 1 an, saugt ab, trocknet und erhält 1,15 g praktish racemische trans-Permethrinsaure ($[\alpha]_D^{25}$ -8,5° (c = 1,9, Chloroform)), die zur Trennung rückgeführt werden kann.

**Beispiel 8**

Man löst 8,8 g D-cis-Permethrinsaure ($[\alpha]_D^{25}$ + 24° (c = 2, Chloroform)), in einer Mischung von 17 ml 10 %igem Natriumhydroxid und 10 ml Wasser, kühlt auf + 10° C ab und fügt 5 ml 2 N-Salzäure hinzu. Man saugt das ausgeschiedene Produkt ab. Auf diese Weise erhält man 2,4 g Produkt ($[\alpha]_D^{25}$ +5,5° (c = 2, Chloroform)). Man gibt zum Filtrat Salzsäure bis pH 1 zu, saugt die erhaltenen Kristalle ab, wäscht zweimal mit je 5 ml Wasser. trocknet und erhält 6,0 g Produkt ($[\alpha]_D^{25}$ +26° (c = 2,1 Chloroform)).

**Beispiel 9**

Man löst 62 g Chrysanthemumsäure, die reich an trans-Isomer ist (cis/trans-Isomerenverhältnis 18:82) und nach Beispiel 3a) hergestellt wurde, in 70 ml 20 %igem Natriumhydroxid bei 70 - 75°C. Man läßt die Lösung auf Raumtemperatur abkühlen und über Nacht stehen. Man saugt dann die ausgeschiedenen Kristalle ab, dampft gründlich ein und löst in 330 ml Wasser. Man fügt zu der erhaltenen Lösung des Na-Salzes 240 ml Petrolether und säuert unter Rühren mit conc. Salzsäure auf pH 1 an. Man trennt dann die organische Phase ab und vermischt die wässerige Phase erneut mit 75 ml Petrolether. Man wäscht die vereinigten organischen Phasen dreimal mit je 100 ml Wasser, trocknet über Natriumsulfat und engt auf ein Viertel ein. Man kühlt danach die Lösung langsam auf 25°C ab und rührt 1 h bei dieser Temperatur. Nach Absaugen einer kleinen Menge an cis-Isomer reicher Kristalle kühlt man die Mutterlauge bis auf -10°C ab. Man saugt die erhaltenen Kristalle erneut ab. Auf diese Weise erhält man 23,1 g D,L-trans-Chrysanthemumsäure mit einer Reinheit von 99,8 %, die analog Beispiel 3b) aufgetrennt werden kann.

**Beispiel 10**

Man regeneriert das Trennmittel aus den wässerigen Lösungen der Salze sowie der Säuren, die durch Aufarbeitung der Mutterlaugen erhalten wurden, wie folgt: Man entfernt die Spuren der organischen Lösungsmittel durch 10 min Kochen und Klärung mit Knochenkohle. Man kühlt dann die Losung auf +15°C ab und macht mit 5 N Natriumhydroxid bis auf pH 11 alkalisch. Man saugt die kristalline Suspension nach 1 h Eiskühlung ab, wäscht dreimal mit Wasser und trocknet. In dieser Weise können die Enantiomeren des N-Benzyl-2-aminobutanols mit einer Ausbeute von 85 % zurückgewonnen werden.

**Patentansprüche**

1. Verfahren zur Gewinnung der optisch aktiven cis- und trans-Isomeren von sehenylcyclopropancarbonsäurederivaten durch Trennung von D,L-cis,trans-, D,L-cis- und D, L-trans-Alkenylcyclopropancarbonsäurederivaten durch

– Umsetzung der entsprechenden Isomerengemische in Form löslicher Alkalimetallsalze mit einem Isomer eines optisch aktiven Amins in einer wäßrigen Lösung unter Ausfällung eines kristallinen diastereomeren Ammoniumsalzes,
– Absaugen dieses diastereomeren Ammoniumsalzes,
– Überführung durch Ansäuern mit einer Mineralsäure in das entsprechende optisch aktive Alkenylcyclopropancarbonsäurederivat,
– Abtrennung dieses Derivats und
– Gewinnung des anderen Isomers oder Isomerengemischs durch Ansäuern des erhaltenen Filtrats mit

einer Mineralsäure sowie gegebenenfalls

– Reinigung des erhaltenen optisch aktiven Isomers der Verbindung der Formel I durch selektive Abscheidung und

– Regenerierung des eingesetzten optisch aktiven Amins, dadurch gekennzeichnet, daß

– (a) Salze von D,L-cis,trans-Vinylcyclopropancarbonsäurederivaten der Formel I

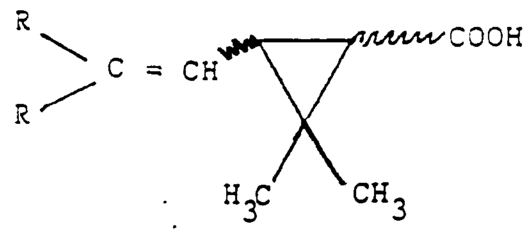

(I)

mit R = $CH_3$ oder Cl
in beliebigem Isomerenverhältnis oder

– (b) durch selektives Lösen in apolaren aromatischen oder aliphatischen Kohlenwasserstoffen als Lösungsmitteln hergestellte reine , Formel I entsprechende D,L-cis- und D,L-trans-Vinylcyclopropancarbonsäurederivate,
die mit Alkalihydroxiden oder Alkalicarbonaten in entsprechende Salze übergeführt wurden,
eingesetzt werden,

– als Amin 2-N-Benzylaminobutanol oder das Hydrochlorid davon verwendet wird und

– die Umsetzung in Wasser oder einem Wasser-Aceton- Gemisch durchgeführt wird.

2. Verfahren nach Anspruch 1, gekennzeichnet durch Verwendung von Benzol, Extraktionsbenzin oder n-Hexan als apolare Lösungsmittel.

3. Verfahren nach Anspruch 1 oder 2 zur Herstellung von Formel I nach Anspruch 1 entsprechenden D-trans-Vinylcyclopropancarbonsäurederivaten, gekennzeichnet durch

– Umsetzung einer wäßrigen Lösung der Salze der racemischen Säure mit einem beliebigen cis,trans-Verhältnis zusammen mit 1,0 bis 1,3 Äquivalent eines Alkalihydroxids mit einer Lösung von D-2-N-Benzylaminobutanol in einem aliphatischen Keton bei 40 bis 60°C in einer zum D-Isomergehalt des Racemats äquivalenten Menge,

– Absaugen des erhaltenen kristallinen Salzes des Formel I nach Anspruch 1 entsprechenden D-trans-Vinylcyclopropancarbonsäurederivats mit D-2-N-Benzylaminobutanol und

– Aufarbeitung analog Anspruch 1.

4. Verfahren nach Anspruch 3, gekennzeichnet durch Anwendung von 1,2 Äquivalent Alkalihydroxid sowie Verwendung von Aceton als alipatisches Keton.

5. Verfahren nach Anspruch 1 oder 2 zur Herstellung von Formel I von Anspruch 1 entsprechenden D-trans-Vinylcyclopropancarbonsäurederivaten, gekennzeichnet durch

– weitere Behandlung der Formel I von Anspruch 1 entsprechenden D,L-cis,trans-Vinylcyclopropancarbonsäurederivate mit aromatischen und aliphatischen Lösungsmitteln bei 25 bis 30 °C unter Rühren und Erhalt der entsprechenden D,L-trans-Vinylcyclopropancarbonsäurederivate

– Umsetzung einer Lösung der erhaltenen D,L-trans-Vinylcyclopropancarbonsäurederivate in Wasser in Gegenwart von 1,0 bis 1,3 Äquivalent eines Alkalihydroxids bei 40 bis 90°C mit einer Lösung von D-2-N-Benzylaminobutanol in Wasser oder einem Aceton-Wasser-Gemisch in einer Menge von 0,4 bis 0,6 Äquivalent,

– Abtrennen des erhaltenen kristallinen Salzes des Formel I von Anspruch 1 entsprechenden-D-trans-Vinylcyclopropancarbonsäurederivats mit D-2-N-Benzylaminobutanol unter Kühlung und

– Aufarbeitung gemäß Anspruch 1.

6. Verfahren nach Anspruch 5, gekennzeichnet durch Verwendung von Benzol, Extraktionsbenzin oder n-Hexan sowie von 0,5 Äquivalent D-2-N-Benzylaminobutanol.

7. Verfahren nach Anspruch 1 oder 2 zur Herstellung von Formel I von Anspruch 1 entsprechenden L-cis-Vinylcyclopropancarbonsäurederivaten, gekennzeichnet durch

– Gewinnung des entsprechenden racemischen cis-Isomers aus dem festen Rückstand aus der Behandlung des Formel I von Anspruch 1 entsprechenden D,L-cis,trans-Vinylcyclopropancarbonsäurederivats mit einem aromatischen oder aliphatischen Kohlenwasserstoff in großer Reinheit,

– Lösen mit 1,0 bis 1,3 Äquivalent eines Alkalihydroxids oder Alkalicarbonats in Wasser,

– Versetzen der erhaltenen Lösung mit einer Lösung von 0,4 bis 0,6 Äquivalent D-2-N-Benzylaminobutanol in einem Aceton-Wasser-Gemisch bei 40 bis 90° C,

– Abtrennen des erhaltenen kristallinen Salzes des Formel I von Anspruch 1 entsprechenden L-cis-Vinylcyclopropancarbonsäurederivats mit D-2-N-benzylaminobutanol nach Abkühlen und

– Aufarbeitung gemäß Anspruch 1.

8. Verfahren nach Anspruch 7, gekennzeichnet durch Verwendung von Benzol oder n-Hexan als Kohlenwasserstoffe und Anwendung von 0,5 Äquivalent D-2-N-Benzylaminobutanol.

9. Verfahren nach einem der Ansprüche 1 bis 4, 7 und 8 zur Trennung der optischen Isomeren der Formel I von Anspruch 1 entsprechenden Vinylcyclopropancarbonsäuren, gekennzeichnet durch

– Gewinnung des D-trans-Isomers aus dem entsprechenden D,L-cis, trans-Vinylcyclopropancarbonsäurederivat analog Anspruch 3,

– Zugabe von L-2-N-Benzylaminobutanol in einer dem Gehalt an L-trans-Vinylcyclopropancarbonsäurederivat des im Filtrat befindlichen L-trans-D,L-cis-Vinylcylopropancarbonsäurederivats entsprechenden Menge zum Filtrat,

– Absaugen des erhaltenen kristallinen Salzes des Formel I von Anspruch 1 entsprechenden L-trans-Vinylcyclopropancarbonsäurederivats mit L-2-N-Benzylaminobutanol,

– Freisetzen des L-trans-Vinylcyclopropancarbonsäurederivats durch Ansäuern mit einer Mineralsäure auf pH 1 und

– Abtrennung des im Filtrat verbliebenen entsprechenden D,L-cis-Vinylcyclopropancarbonsäurederivats gemäß Anspruch 7.

10. Verfahren nach einem der Ansprüche 1 bis 6 und 9 zur Herstellung der Formel I von Anspruch 1 entsprechenden L-trans-Vinylcyclopropancarbonsäure-Enantiomeren, gekennzeichnet durch
Verwendung von L-2-N-Benzylaminobutanol als Diastereomerenpaarbildner.

11. Verfahren nach einem der Ansprüche 1, 2, 7 und 8 zur Herstellung der Formel I von Anspruch 1 entsprechenden D-cis-Vinylcyclopropancarbonsäure-Enantiomeren, gekennzeichnet durch
Verwendung von L-2-N-Benzylaminobutanol als Diastereomerenpaarbildner.

12. Verfahren nach einem der Ansprüche 1 bis 11, gekennzeichnet durch
weitere Reinigung der erhaltenen, Formel I von Anspruch 1 entsprechenden optisch aktiven Vinylcyclopropancarbonsäurederivate durch selektive Abscheidung durch

– Ansäuren einer Lösung eines Alkalisalzes des entsprechenden Isomers in Wasser durch Zugabe einer Mineralsäure bei 0 bis 10°C in einer Menge, die kleiner ist als die aufgrund der optischen Reinheit der betreffenden Verbindung berechnete äquivalente Menge,

– Abtrennen des ausgeschiedenen Vinylcyclopropancarbonsäurederivats und

– Ansäuern des Filtrats mit einer Mineralsäure auf pH 1 und Abtrennung des als zweite Fraktion ausgeschiedenen Vinylcyclopropancarbonsäurederivats,

– wobei bei Aufarbeitung von cis-Vinylcyclopropancarbonsäurederivaten die erhaltene erste Fraktion praktisch ein Racemat und die zweite Fraktion die optisch reine Säure und

bei Aufarbeitung von trans-Vinylcyclopropancarbonsäurederivaten die erhaltene erste Fraktion die reine Säure und die zweite Fraktion praktisch ein Racemat darstellen.

13. Verfahren nach einem der Ansprüche 1 bis 8, gekennzeichnet durch praktisch quantitative Durchführung der Herstellung von racemischen cis- und racemischen trans-Isomerengemischen mit beliebigem Isomerenverhältnis bzw. von reinen D,L-cis- und D,L-trans-Isomeren aus D,L-cis,trans-Vinylcyclopropancarbonsäurederivat-Isomeren mit bestimmtem Isomerenverhältnis durch

– erneute Verwendung von regenerierten Vinylcyclopropancarbonsäurederivaten, die durch Abdampfen des aromatischen bzw. aliphatischen Kohlenwasserstoff-Lösungsmittels aus Derivate der Vinylcyclopropancarbonsäure enthaltenden Lösungen erhalten wurden, und

– Gewinnung der optischen Isomeren nach dem Verfahren der Anspruche 1 bis 8.

14. Verfahren nach einem der Ansprüche 1 bis 11, gekennzeichnet durch

– Regenerierung des jeweiligen eingesetzten 2-N-Benzylaminobutanols durch Klären der nach dem Abtrennen der optisch aktiven Vinylcyclopropancarbonsäurederivate durch Entfernen der organischen Lösungsmittel entstehenden sauren wäßrigen Lösungen und Alkalischmachen auf pH 11 in großer Reinheit und

– Rückführung zur Wiederverwendung.

**Revendications**

1. Procédé d'obtention des isomères cis et trans optiquement actifs de dérivés d'acides alcénylcyclopro-

panecarboxyliques par séparation de dérivés d'acides D,L-cis,trans, D,L-cis et D,L-trans-alcénylcyclopropanecarboxyliques par

– réaction du mélange d'isomères correspondants sous forme de sels de métaux alcalins solubles avec un isomère d'une amine optiquement active dans une solution aqueuse avec précipitation d'un sel d'ammonium diastéréomère cristallin,
– filtration par succion de ce sel d'ammonium diastéréomère,
– transformation par acidification avec un acide minéral en le dérivé d'acide alcoylènecyclopropane carboxylique optiquement actif correspondant,
– séparation de ce dérivé, et
– obtention de l'autre isomère ou mélange d'isomères par acification du filtrat obtenu avec un acide minéral, ainsi que le cas échéant
– purification de l'isomère optiquement actif obtenu du composé de formule I par une séparation sélective, et
– régénération de l'amine optiquement active utilisée, caractérisé en ce que
– on utilise des sels de dérivés d'acides D,L-cis,trans-vinylcyclopropanecarboxyliques de formule I

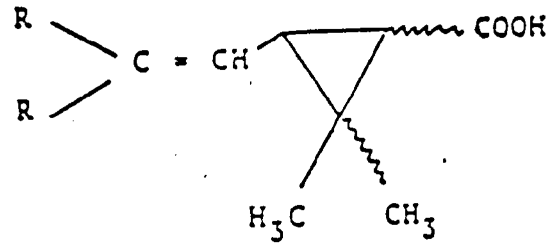

(I)

avec R = $CH_3$ ou Cl
dans un rapport d'isomères quelconque ou
– (b) par dissolution sélective dans des hydrocarbures aromatiques ou aliphatiques apolaires comme solvants on utilise des dérivés d'acides D,L-cis et D,L-trans-vinylcyclopropanecarboxyliques correspondants à la formule I préparés sous forme pure, qui ont été transformés avec des hydroxydes alcalins ou des carbonates alcalins en les sels correspondants,
– on utilise comme amine le 2-N-benzylaminobutanol ou son chlorhydrate, et
– on conduit la réaction dans l'eau ou dans un mélange eau-acétone.

2. Procédé selon la revendication 1, caractérisé par l'utilisation de benzène, essence d'extraction ou n-hexane comme solvant apolaire.

3. Procédé selon les revendications 1 ou 2 de préparation de dérivés d'acide D-trans-vinylcyclopropanecarboxylique correspondant à la formule I selon la revendication 1, caractérisé par

– réaction d'une solution aqueuse des sels de l'acide racémique dans un rapport cis,trans quelconque avec de 1,0 à 1,3 équivalent d'un hydroxyde alcalin avec une solution de D-2-N-benzylaminobutanol dans une cétone aliphatique à 40 à 60°C dans une quantité équivalant à la teneur en isomère D du racémate,
– filtration par succion du sel cristallin obtenu du dérivé d'acide D-trans-vinylcyclopropanecarboxylique correspondant à la formule I selon la revendication 1 avec le D-2-N-benzylaminobutanol et purification de manière analogue à la revendication 1.

4. Procédé selon la revendication 3, caractérisé par l'utilisation de 1,2 équivalent d'hydroxyde alcalin ainsi que par l'utilisation d'acétone comme cétone aliphatique.

5. Procédé selon les revendications 1 ou 2 de préparation de dérivés d'acide D-trans-vinylcyclopropanecarboxylique correspondant à la formule I de la revendication 1 , caractérisé par

– traitement ultérieur de dérivés d'acide D,L-cis,trans-vinylcyclopropanecarboxylique correspondant à la formule I de la revendication 1 avec des solvants aromatiques et aliphatiques à 25 à 30°C avec agitation et obtention des dérivés d'acide D,L-trans-vinylcyclopropanecarboxylique correspondants,
– réaction d'une solution des dérivés d'acide D,L-trans-vinylcyclopropanecarboxylique obtenus dans l'eau en présence de 1,0 à 1,3 équivalent d'un hydroxyde alcalin à 40 à 90°C avec une solution de D-2-N-benzylaminobutanol dans l'eau ou un mélange acétone-eau en une quantité de 0,4 à 0,6 équivalent,
– séparation du sel cristallin obtenu du dérivé d'acide D-trans-vinylcyclopropanecarboxylique correspondant à la formule I de la revendication 1 avec le D-2-N-benzylaminobutanol en refroidissant et
– purification selon la revendication 1.

6. Procédé selon la revendication 5, caractérisé par l'utilisation de benzène, d'essence d'extraction ou de n-hexane ainsi que de 0,5 équivalent de D-2-N-benzylaminobutanol.

7. Procédé selon les revendications 1 ou 2 de préparation de dérivés d'acide L-cis-vinylcyclopropanecarboxylique correspondant à la formule I de la revendication 1, caractérisé par
– obtention de l'isomère cis racémique correspondant à partir du résidu solide provenant du traitement du dérivé d'acide D,L-cis,trans-vinylcyclopropanecarboxylique correspondant à la formule I de la revendication 1 avec un hydrocarbure aromatique ou aliphatique avec une grande pureté,
– dissolution avec de 1,0 à 1,3 équivalent d'un hydroxyde alcalin ou carbonate alcalin dans l'eau,
– mélange de la solution obtenue avec une solution de 0,4 à 0,6 équivalent de D-2-N-benzylaminobutanol dans un mélange d'acétone et d'eau à 40 à 90°C.
– séparation du sel cristallin obtenu du dérivé d'acide L-cis-vinylcyclopropanecarboxylique correspondant à la formule I de la revendication 1 avec le D-2-N-aminobutanol après refroidissement et purification selon la revendication 1.
8. Procédé selon la revendication 7, caractérisé par l'utilisation de benzène ou de n-hexane comme hydrocarbures et l'application de 0,5 équivalent de D-2-N-benzylaminobutanol.
9. Procédé selon l'une des revendications 1 à 4, 7 et 8 de séparation des isomères optiques d'acides vinylcyclopropanecarboxyliques correspondant à la formule I de la revendication 1, caractérisé par
– obtention de l'isomère D-trans à partir du dérivé d'acide D,L-cis,trans-vinylcyclopropanecarboxylique correspondant de manière analogue à la revendication 3,
– addition, dans le filtrat de L-2-N-benzylaminobutanol dont la teneur en dérivé d'acide L-trans-vinylcyclopropane carboxylique correspond à celle du dérivé d'acide L-trans-D,L-cis-vinylcyclopropanecarboxylique dans le filtrat.
– filtration par succion du sel cristallin obtenu du dérivé d'acide L-trans-vinylcyclopropanecarboxylique correspondant à la formule I de la revendication 1 avec le L-2-N-benzylaminobutanol,
– libération du dérivé d'acide L-trans-vinylcyclopropanecarboxylique par acidification avec un acide minéral à pH 1 et
– séparation du dérivé d'acide D,L-cis-vinylcyclopropanecarboxylique correspondant resté dans le filtrat selon la revendication 7.
10. Procédé selon l'une des revendications 1 à 6 et 9 de préparation d'énantiomères d'acide L-trans-vinylcyclopropanecarboxylique correspondant à la formule I de la revendication 1, caractérisé par utilisation de L-2-N-benzylaminobutanol comme formateur de paires de diastéréomères.
11. Procédé selon l'une des revendications 1, 2, 7 et 8 de préparation d'énantiomères d'acide D-cis-vinylcyclopropanecarboxylique correspondant à la formule I de la revendication 1, caractérisé par utilisation de L-2-N-benzylaminobutanol comme formateur de paires de diastéréomères.
12. Procédé selon l'une des revendications 1 à 11, caractérisé par une purification plus poussée des dérivés d'acide vinylcyclopropanecarboxylique optiquement actifs obtenus, correspondant à la formule I de la revendication 1, par séparation sélective par
– acidification d'une solution d'un sel alcalin de l'isomère correspondant dans l'eau par addition d'un acide minéral à 0 à 10°C en une quantité qui est plus faible que la quantité équivalente calculée sur la base de la pureté optique du composé en question,
– séparation du dérivé d'acide vinylcyclopropanecarboxylique précipité, et
– acidification du filtrat avec un aride minéral à pH 1 et séparation du dérivé d'aride cyclopropanerarboxylique précipité sous forme de seconde fraction,
– où dans la purification des dérivés d'acide cis-vinylcyclopropanecarboxylique la première fraction obtenue représente pratiquement un racémate et la seconde fraction l'acide optiquement pur et dans la purification des dérivés d'acide trans-vinylcyclopropanecarboxylique la première fraction obtenue représente l'acide pur et la seconde fraction en pratique un racémate.
13. Procédé selon l'une des revendications 1 à 8, caractérisé par une réalisation pratiquement quantitative de la préparation de mélanges d'isomères racémiques cis et racémiques trans avec un rapport d'isomères quelconque, ou selon les cas d'isomères D,L-cis et D,L-trans purs à partir d'isomères de dérivés d'acide D,L-cis,trans-vinylcyclopropanecarboxylique avec un rapport d'isomères déterminé par
– réutilisation de dérivés d'acide vinylcyclopropanecarboxylique régénérés, qui ont été obtenus par évaporation du solvant hydrocarboné aromatique ou selon les cas aliphatique et des solutions contenant l'acide vinylcyclopropanecarboxylique, et
– obtention des isomères optiques selon le procédé des revendications 1 à 8.
14. Procédé selon l'une des revendications 1 à 11, caractérisé par
– régénération du 2-N-benzylaminobutanol utilisé à chaque fois par clarification des solutions aqueuses acides apparues après séparation des dérivés d'acide vinylcyclopropanecarboxylique optiquement actifs par retrait des solvants organiques et alcalinisation à pH 11 avec une grande pureté et
– recyclage pour la réutilisation.

## Claims

1. A process for obtaining the optically active cis- and trans-isomers of alkenylcyclopropane carboxylic acid derivatives by separating D,L-cis,trans-, D,L-cis- and D,L-trans-alkenylcyclopropanecarboxylic acid derivates by

– reacting the corresponding isomer mixtures in the form of soluble alkali metal salts with an isomer of an optically active amine in an aqueous solution, with precipitation of a crystalline diastercomeric ammonium salt,

– suction filtering this diastereomeric ammonium salt,

– conversion, by acidification with an inorganic acid, into the corresponding optically active alkenylcyclopropane-carboxylic acid derivative,

– separating this derivative and

– recovering the other isomer or isomer mixture by acidifying the resulting filtrate with an inorganic acid and optionally

– purifying the resulting optically active isomer of the compound of formula I by selective precipitation and

– regenerating the optically active amine used, characterised in that

– (a) salts of D,L-cis,trans-vinylcyclopropanecarboxylic acid derivates of formula I

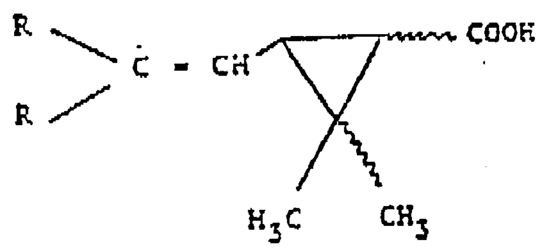

(I)

wherein R = $CH_3$ or Cl

in any desired isomeric ratio are used, or

– (b) pure D,L-cis- and D,L-trans-vinylcyclopropane-carboxylic acid derivatives corresponding to formula I and prepared by selective dissolving in apolar aromatic or aliphatic hydrocarbons as solvents, converted into corresponding salts with alkali metal hydroxides or alkali metal carbonates are used,

– 2-N-benzylaminobutanol or the hydrochloride thereof is used as amine and

– the reaction is carried out in water or a mixture of water and acetone.

2. A method according to claim 1, characterized by the use of benzene, extraction benzene or n-hexane as apolar solvents.

3. A method according to claim 1 or 2 for preparing D-trans-vinylcyclopropane carboxylic acid derivatives corresponding to formula I of claim 1, characterized by

– reacting an aqueous solution of the salts of the racemic acid having any cis-trans-ratio together with 1.0 to 1.3 equivalents of an alkali hydroxide with a solution of D-2-N-benzylamiobutanol in an aliphatic ketone at 40 to 60°C in an amount equilavent to the content of D-isomer of the racemate,

– suction filtering the obtained crystalline salt of the D-trans-vinylcyclopropane carboxylic acid derivative corresponding to formula I of claim 1 with D-2-N-benzylaminobutanol, and

– further treatment analogous to claim 1.

4. A method according to claim 3, characterized by the use of 1,2 equivalents of an alkali metal hydroxide, and the use of acetone as an aliphatic ketone.

5. A method according to claim 1 or 2 for preparing D-trans-vinylcyclopropane carboxylic acid derivatives corresponding to formula I of claim 1, characterized by

– further treating D,L-cis,trans-vinylcyclopropane carboxylic acid derivatives corresponding to formula I of claim 1 with aromatic and aliphatic solvents at 25 to 30°C with stirring and obtaining the corresponding D,L-trans-vinylcyclopropane carboxylic acid derivatives,

– reacting a solution of the obtained D,L-transvinylcyclopropane carboxylic acid derivatives in water in the presence of 1.0 to 1.3 equivalents of an alkali hydroxide at 40 to 90°C with a solution of D-2-N-benzylaminobutanol in water or an acetone-water mixture in an amount of 0.4 to 0.6 equivalent,

– separating the obtained crystalline salt of the D-trans-vinylcyclopropane carboxylic acid derivative according to formula I of claim 1 with D-2-N-benzylaminobutanol with cooling, and

– further treatment analogous to claim 1.

6. A method according to claim 5, characterized by the use of benzene, extraction naphtha or n-hexane and of 0.5 equivalent of D-2-N-benzylaminobutanol.

7. A method according to claim 1 or 2 for preparing L-cis-vinylcyclopropane carboxylic acid derivatives corresponding to formula I of claim 1, characterized by

– obtaining the correponding racemic cis-isomer in high purity from the solid residue resulting from the treatment of the D,L-cis,trans-vinylcyclopropane carboxylic acid derivative corresponding to formula I of claim 1 with an aromatic or aliphatic hydrocarbon,

– dissolving in water together with 1.0 to 1.3 equivalents of an alkali hydroxide or alkali carbonate,

– adding a solution of 0.4 to 0.6 equivalent of D-2-N-benzylaminobutanol in an acetone-water mixture to the obtained solution at 40 to 90°C,

– separating the obtained crystalline salt of the L-cis-vinylcyclopropane carboxylic acid derivative corresponding to formula I of claim 1 with D-2-N-benzylaminobutanol after cooling, and

– futher treatment according to claim 1.

8. A method according to claim 7, characterized by the use of benzene or n-hexane as hydrocarbons, and the use of 0.5 equivalent of D-2-N-benzylaminobutanol.

9. A method according to one of claims 1 to 4, 7 and 8 for separating the optical isomers of vinylcyclopropane carboxylic acids corresponding to formula I of claim 1, characterized by

– obtaining the D-trans-isomer from the corresponding D,L-cis,trans vinylcyclopropane carboxylic acid derivative analogously to claim 3,

– adding L-2-N-benzylaminobutanol in an amount corresponding to the content of L-trans-vinylcyclopropane carboxylic acid derivative of the L-trans-D,L-cis-vinylcyclopropane carboxylic acid derivatives present in the filtrate, to the filtrate,

– suction filtering the obtained crystalline salt of the L-trans-vinylcyclopropane carboxylic acid derivative corresponding to formula I of claim 1 with L-2-N-benzylaminobutanol,

– liberating the L-trans-vinylcyclopropane carboxylic acid derivative by acidifying with a mineral acid to pH 1, and

– separating the corresponding D,L-cis-vinylcyclopropane carboxylic acid derivative remaining in the filtrate according to claim 7.

10. A method according to one of claims 1 to 6 and 9 for preparing L-trans-vinylcyclopropane carboxylic acid enantiomers corresponding to formula I of claim 1, characterized by the use of L-2-N-benzylaminobutanol for forming the pairs of disastereomers.

11. A method according to one of claims 1, 2, 7, and 8 for preparing D-cis-vinylcyclopropane carboxylic acid enantiomers corresponding to formula I of claim 1, characterized by the use of L-2-N-benzylaminobutanol for forming the pairs of diasteromers.

12. A method according to one of claims 1 to 11, characterized by futher purifying the obtained optically active vinylcyclopropane carboxylic acid derivatives corresponding to formula I of claim 1 by means of selective precipitation by

– acidifying a solution of an alkali metal salt of the corresponding isomer in water by adding a mineral acid at 0 to 10°C in an amount less than the equivalent amount calculated on the basis of the optical purity of the corresponding compound,

– separating the precipitated vinylcyclopropane carboxylic acid derivative, and

– acidifying the filtrate with a mineral acid to pH 1 and separating the vinylcyclopropane carboxylic acid derivative precipitated as second fraction,

– wherein when cis-vinylcyclopropane carboxylic acid derivatives are treated, the first obtained fraction practically represents a racemate, and the second fraction the optically pure acid, and

– when trans-vinylcyclopropane carboxylic acid derivatives are treated, the first fraction obtained represents the pure acid and the second fraction practically represents a racemate.

13. A method according to one of claims 1 to 8, characterized by a practically quantitative execution of preparing racemic cis- and racemic trans-isomer mixtures having any isomer ratio or of pure D,L-cis- and D,L-transisomers, respectively, of isomers of D,L-cis,trans-vinylcyclopropane carboxylic acid derivatives having a defined isomer ratio by

– reusing regenerated vinylcyclopropane carboxylic acid derivatives obtained by evaporating the aromatic or aliphatic hydrocarbon solvent, respectively, from solutions containing vinylcyclopropane carboxylic acid derivatives, and

– obtaining the optical isomers according to the method of claims 1 to 8.

14. A method according to one of claims 1 to 11, characterized by

– regenerating the respective used 2-N-benzylamino-butanol by clearing the acidic aqueous solutions resulting after separation of the optically active vinylcyclopropane carboxylic acid derivatives by removing the organic solvents, and alkalizing to pH 11 in high purity, and

– recycling for reuse.